# EUROPEAN PATENT SPECIFICATION

(11) **EP 2 453 872 B1**
(45) Date of publication and mention of the grant of the patent: **21.08.2013**
(21) Application number: 10735130.6
(22) Date of filing: 15.07.2010
(51) Int. Cl.: A61K 9/00, A61K 31/335

(54) **OLOPATADINE NASAL SPRAY REGIMEN FOR CHILDREN**
OLOPATADIN-NASENSPRAYBEHANDLUNG FÜR KINDER
SCHÉMA POSOLOGIQUE POUR L'ADMINISTRATION D'OLOPATADINE EN PULVÉRISATION NASALE À DES ENFANTS

(30) Priority: 17.07.2009 US 226469 P
(43) Date of publication of application: 23.05.2012
(73) Proprietor: Alcon Research, Ltd., Fort Worth, TX 76134-2099 (US)
(72) Inventor: WALL, Michael, G., Fort Worth Texas 76109 (US)
(74) Representative: Best, Michael
(86) International application number: PCT/US2010/042085
(87) International publication number: WO 2011/008923

(56) References cited:
- US-A1- 2007 142 458

## Description

### BACKGROUND OF THE INVENTION

### Field of the Invention

The present invention relates to topical formulations used for treating allergic and inflammatory diseases. More particularly, the present invention relates to formulations of olopatadine and their use for treating and/or preventing allergic or inflammatory disorders of the nose in children.

### Description of the Related Art

As taught in U.S. Patent Nos. 4,871,865 and 4,923,892, both assigned to Burroughs Wellcome Co. ("the Burroughs Wellcome Patents"), certain carboxylic acid derivatives of doxepin, including olopatadine (chemical name: Z-11-(3-dimethylaminopropylidene)-6,11-dihydrodibenz[b,e]oxepine-2-acetic acid), have antihistamine and antiasthmatic activity. These two patents classify the carboxylic acid derivatives of doxepin as mast cell stabilizers with antihistaminic action because they are believed to inhibit the release of autacoids (i.e., histamine, serotonin, and the like) from mast cells and to inhibit directly histamine's effects on target tissues. The Burroughs Wellcome Patents teach various pharmaceutical formulations containing the carboxylic acid derivatives of doxepin, including nasal spray and ophthalmic formulations. See, for example, Col. 7, lines 7 - 26, and Examples 8 (H) and 8 (I) of the '865 patent.

U.S. Patent No. 5,116,863, assigned to Kyowa Hakko Kogyo Co., Ltd., ("the Kyowa patent"), teaches that acetic acid derivatives of doxepin and, in particular, olopatadine, have anti-allergic and anti-inflammatory activity. Olopatadine is the *cis* form of the compound having the formula: 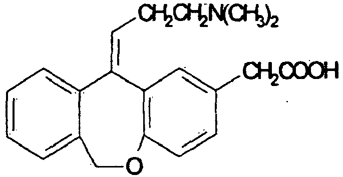 Medicament forms taught by the Kyowa patent for the acetic acid derivatives of doxepin include a wide range of acceptable carriers; however, only oral and injection administration forms are mentioned.

U.S. Patent No. 5,641,805, assigned to Alcon Laboratories, Inc. and Kyowa Hakko Kogyo Co., Ltd., teaches topical ophthalmic formulations containing olopatadine for treating allergic eye diseases. According to the '805 patent, the topical formulations may be solutions, suspensions or gels. The formulations contain olopatadine, an isotonic agent, and "if required, a preservative, a buffering agent, a stabilizer, a viscous vehicle and the like." See Col. 6, lines 30 - 43. "[P]olyvinyl alcohol, polyvinylpyrrolidone, polyacrylic acid or the like" are mentioned as the viscous vehicle. See Col. 6, lines 55-57.

PATANOL^{®} (olopatadine hydrochloride ophthalmic solution) 0.1% is a commercially available olopatadine product for ophthalmic use. According to its labelling information, it contains olopatadine hydrochloride equivalent to 0.1% olopatadine, 0.01% benzalkonium chloride, and unspecified amounts of sodium chloride, dibasic sodium phosphate, hydrochloric acid and/or sodium hydroxide (to adjust pH) and purified water.

PATANASE^{®} (olopatadine hydrochloride nasal spray) 0.6% olopatadine is a commercially available olopatadine product for nasal use. According to its labelling information, it contains olopatadine hydrochloride equivalent to 0.1% olopatadine, 0.01% benzalkonium chloride, and unspecified amounts of sodium chloride, edetate disodium, dibasic sodium phosphate, hydrochloric acid and/or sodium hydroxide (to adjust pH) and purified water. United State Patent Publication no. 20070142458 discloses olopatadine nasal spray formulations.

Unexpectedly, it has been discovered that olopatadine nasal spray can provide improved alleviation of allergy symptoms for children when the nasal spray is administered in smaller doses relative to larger doses, which are traditionally administered to adults.

### Summary of the Invention

The present invention provides a method of administering olopatadine nasal spray that is particularly desirable for children. The present invention also provides a pharmaceutical product desirable for administering the nasal spray to children. The formulations of the present invention are aqueous solutions that comprise approximately 0.6 % olopatadine and are effective as products for treating allergic or inflammatory disorders of the nose.

According to the method, a first amount of olopatadine is administered to nostrils of a child during a first time period. A second amount of olopatadine is then administered to the nostrils of the child during a second time period. The first amount and second amount are each at least about 0.9 mg but no more than about 1.5 mg. Moreover, the first time period and the second time period of administration are separated by an intermediate time period that is at least four hours but is less than twenty four hours. Typically, the first time period and second time period are both less than two minutes. The term "child" can mean any individual under the age of 12, but preferably means an individual that is at least 2 years of age, more preferably at least 6 years of age but is no more than 11 years of age or less than 12 years of age. The first amount and the second amount are typically administered from a nasal spray bottle to the nostrils. Additionally, the first amount and second amount are typically each delivered to the nostrils of the child using a single spray from the nasal spray bottle in each nostril. This first amount and second amount of olopatadine can each be administered on a daily basis (i.e., the first amount and second amount can each be administered in consecutive 24 hour periods) for multiple days (e.g., at least 15, 30, 45 or more days). For example, the amounts can be delivered daily through an allergy season.

The olopatadine pharmaceutical product typically comprises a nasal sprayer containing an olopatadine nasal spray composition and instructions for administration of the nasal spray to a child provided therewith. Administration of the nasal spray according to the instructions results in administration of olopatadine in accordance with the following regimen:
i) administration of a first amount of olopatadine to nostrils of a child during a first time period wherein the first time period is less than two minutes and wherein the child is at least 4 years of age but is no more than 11 years of age or less than 12 years of age and wherein the first amount is at least about 0.9 mg but is no more than about 1.5 mg; and
ii) administration of a second amount of olopatadine to the nostrils of the child during a second time period wherein the second time period is less than two minutes and wherein the first time period and the second time period are separated by an intermediate time period that is at least four hours but is less than twenty four hours and the second time period is less than two minutes and wherein the second amount is at least about 0.9 mg but is no more than about 1.5 mg.
Again, this first amount and second amount of olopatadine can be administered on a daily basis for multiple days.

In a preferred embodiment, the nasal spray composition typically includes or consists essentially of the following:
a) 0.54 - 0.62 % (w/v) olopatadine free base or an equivalent amount of a pharmaceutically acceptable salt of olopatadine;
b) a phosphate salt in an amount equivalent to 0.2 - 0.8 % (w/v) dibasic sodium phosphate, wherein the phosphate salt selected from the group consisting of monobasic sodium phosphate; dibasic sodium phosphate; tribasic sodium phosphate; monobasic potassium phosphate; dibasic potassium phosphate; and tribasic potassium phosphate;
c) 0.3 - 0.6 % (w/v) NaCl;
d) a pH-adjusting agent in an amount sufficient to cause the composition to have a pH of 3.6 - 3.8;
e) 0.005 - 0.015 % (w/v) benzalkonium chloride;
f) 0.005 - 0.015 % (w/v) edetate disodium; and
g) water.

### Detailed Description of the Invention

The present invention is predicated upon the unexpected discovery that, for children, lower doses of olopatadine nasal spray can perform better in alleviating allergy symptoms, particularly nasal allergy symptoms, than higher doses of olopatadine. This is particularly the case for children of ages four to eleven. Accordingly, a regimen for nasal administration of olopatadine nasal spray to children has been developed.

Unless indicated otherwise, all component amounts are presented on a % (w/v) basis and all references to amounts of olopatadine are to olopatadine free base.

Olopatadine is a known compound that can be obtained by the methods disclosed in U.S. Patent No. 5,116,863. The solution formulations of the present invention contain 0.54 - 0.72% olopatadine. Preferably, the solution formulations contain 0.6% olopatadine.

Olopatadine has both a carboxylic functional group (pKa₁ = 4.18) and a tertiary amino group (pKa₂ = 9.79). It exists in different ionic forms depending upon the pH of the solution. Olopatadine exists predominantly as a zwitterion in the pH range between the two pKa values with a negatively-charged carboxylic group and a positively-charged tertiary amino group. The iso-electric point of the olopatadine zwitterion is at pH 6.99. At a pH lower than pka₁, cationic olopatadine (with ionized tertiary amino group) is dominant. At a pH higher than pKa₂, anionic olopatadine (with ionized carboxylic group) is dominant.

### Acid-Base Equilibrium of Olopatadine

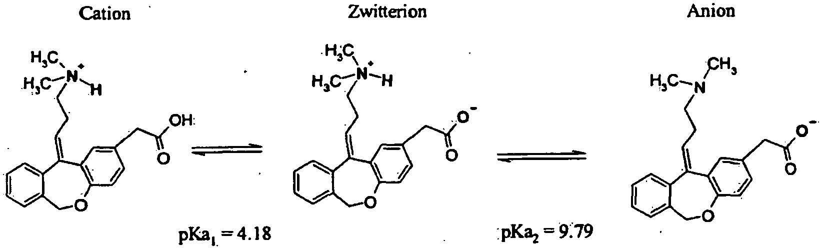

In many zwitterionic molecules, such as various amino acids, intra-molecular ionic interactions are not significant or do not exist. But the structure of olopatadine is such that intra-molecular interactions exist and are significant, possibly due to the distance and bonding angle between the oppositely charged functional groups. This interaction effectively reduces the ionic and dipole character of the molecule. The net effect of the intra-molecular interactions between the oppositely charged functional groups is the reduction of aqueous solubility of olopatadine. Olopatadine has the pH-solubility profile shown in Figures 1A (theoretical) and 1B (obtained using phosphate buffer).

Generally, olopatadine will be added in the form of a pharmaceutically acceptable salt. Examples of the pharmaceutically acceptable salts of olopatadine include inorganic acid salts such as hydrochloride, hydrobromide, sulfate and phosphate; organic acid salts such as acetate, maleate, fumarate, tartrate and citrate; alkali metal salts such as sodium salt and potassium salt; alkaline earth metal salts such as magnesium salt and calcium salt; metal salts such as aluminum salt and zinc salt; and organic amine addition salts such as triethylamine addition salt (also known as tromethamine), morpholine addition salt and piperidine addition salt. The most preferred form of olopatadine for use in the solution compositions of the present invention is the hydrochloride salt of (Z)-11-(3-dimethylaminopropylidene)-6,11-dihydro-dibenz-[b,e]oxepin-2-acetic acid. When olopatadine is added to the compositions of the present invention in this salt form, 0.665% olopatadine hydrochloride is equivalent to 0.6% olopatadine free base. Preferably the compositions of the present invention comprise approximately 0.665% olopatadine hydrochloride.

In addition to olopatadine, the aqueous solution compositions of the present invention comprise a phosphate salt. The phosphate salt not only helps maintain the pH of the compositions within the targeted pH range of 3.5 - 3.95 by contributing to the buffer capacity of the compositions, but also helps solubilize olopatadine. Suitable phosphate salts for use in the compositions of the present invention include monobasic sodium phosphate, dibasic sodium phosphate, tribasic sodium phosphate, monobasic potassium phosphate, dibasic potassium phosphate, and tribasic potassium phosphate. The most preferred phosphate salt is dibasic sodium phosphate. The compositions of the present invention comprise an amount of phosphate salt equivalent (on an osmolality contribution basis) to 0.2 - 0.8 %, preferably 0.3 - 0.7 %, and most preferably 0.4 - 0.6 % of dibasic sodium phosphate. In a preferred embodiment, the phosphate salt is dibasic sodium phosphate at a concentration of 0.4 - 0.6 % (w/v). In a most preferred embodiment, the compositions contain 0.5 % (w/v) dibasic sodium phosphate.

Phosphate buffer is commonly used in aqueous pharmaceutical compositions formulated near neutral pH. Phosphate buffer (pKa₁ = 2.12, pKa₂ = 7.1, pKa₃ = 12.67) would not normally be chosen for an aqueous composition with a target pH range of 3.5 - 3.95 because it has low buffer capacity in that region Other buffering agents are commonly used in aqueous pharmaceutical compositions, including acetate, citrate and borate buffers, but are not suitable for use in the topical nasal compositions of the present invention. Borate buffers are not suitable because they do not have any significant buffer capacity in the pH range 3.5 - 3.95. Though acetate and citrate buffers have buffer capacity in this region, they are not preferred because they have the potential to cause irritation to nasal mucosal tissues and undesirable taste and/or smell.

In addition to olopatadine and phosphate salt, the compositions of the present invention comprise sodium chloride as a tonicity-adjusting agent. The compositions contain sodium chloride in an amount sufficient to cause the final composition to have a nasally acceptable osmolality, preferably 240 - 350 mOsm/kg. Most preferably, the amount of sodium chloride in the compositions of the present invention is an amount sufficient to cause the compositions to have an osmolality of 260 - 330 mOsm/kg. In a preferred embodiment, the compositions contain 0.3 - 0.6 % sodium chloride. In a more preferred embodiment, the compositions contain 0.35 - 0.55 % sodium chloride, and in a most preferred embodiment, the compositions contain 0.35 - 0.45 % sodium chloride.

The compositions of the present invention also contain a pharmaceutically acceptable pH-adjusting agent. Such pH-adjusting agents are known and include, but are not limited to, hydrochloric acid (HCl) and sodium hydroxide (NaOH). The compositions of the present invention preferably contain an amount of pH-adjusting agent sufficient to obtain a composition pH of 3.5 - 3.95, and more preferably, a pH of 3.6 - 3.8.

In one embodiment, the aqueous compositions of the present invention consist essentially of olopatadine, phosphate buffer, sodium chloride, a pH-adjusting agent, and water, and have a pH from 3.5 - 3.95. These compositions can be manufactured as sterile compositions and packaged in multi-dose, pressurized aerosol containers to avoid microbial contamination. In another embodiment, the aqueous compositions of the present invention contain a preservative and a chelating agent such that the compositions pass United States Pharmacopeia/National Formulary XXX criteria for antimicrobial effectiveness, and more preferably the Pharm. Eur. 5th Edition criteria for antimicrobial preservation (Pharm. Eur. B preservative effectiveness standard). Suitable preservatives include p-hydroxybenzoic acid ester, benzalkonium chloride, benzododecinium bromide, and the like. Suitable chelating agents include sodium edetate and the like. The most preferred preservative ingredient for use in the compositions of the present invention is benzalkonium chloride ("BAC"). The amount of benzalkonium chloride is preferably 0.005 - 0.015 %, and more preferably 0.01 %. The most preferred chelating agent is edetate disodium ("EDTA"). The amount of edetate disodium in the compositions of the present invention is preferably 0.005 - 0.015 %, and more preferably 0.01 %.

The aqueous solution compositions of the present invention do not contain a polymeric ingredient intended to enhance the solubility of olopatadine or the physical stability of the solution. For example, the compositions of the present invention do not contain polyvinylpyrrolidone, polystyrene sulfonic acid, polyvinyl alcohol, polyvinyl acrylic acid, hydroxypropylmethyl cellulose, sodium carboxymethyl cellulose or xanthan gum.

The compositions of the present invention are preferably packaged in opaque plastic containers. A preferred container is a high-density polyethylene container equipped with a nasal spray pump. Preferably, the package is designed to provide the spray characteristics described in commonly-assigned, co-pending, U.S. Patent Application Publication No. 2006/0110328.

The present invention also relates to a method of treating allergic rhinitis comprising topically administering to the nasal cavities a composition containing approximately 0.6 % olopatadine, phosphate buffer, sodium chloride, a pH-adjusting agent, and water. The compositions optionally contain one or more preservative ingredients. Preferably, the compositions are administered such that 600 mcg of olopatadine (e.g., 600/mcg per 100 microliter spray x one spray) is delivered to each nostril twice per day.

Example1 below provides a preferred olopatadine nasal spray composition.

### Example 1: Topically Administrable Nasal Solution

**Table 1**

| Ingredient | Amount (%, w/v) |
|---|---|
| Olopatadine Hydrochloride | 0.665 ^{a} |
| Benzalkonium Chloride | 0.01 |
| Edetate Disodium, Dihydrate | 0.01 |
| Sodium Chloride | 0.41 |
| Dibasic Sodium Phosphate, Anhydrous | 0.5 |
| Hydrochloric Acid and/or Sodium Hydroxide | Adjust to pH 3.7 ± 0.1 |
| Purified Water | qs to 100 |

| | |
|---|---|
| ^{a} 0.665% w/v olopatadine hydrochloride (665 mcg/100 microliter spray) is equivalent to 0.6% w/v olopatadine as base (600 mcg/100 microliter spray). | |

An exemplary compounding procedure for the nasal composition shown in Table 1 is described as below.
1. Tare a suitable compounding vessel with magnetic stir bar. Add approximately 80% of the batch weight of purified water.
2. While stirring, add dibasic sodium phosphate (anhydrous), sodium chloride, edetate disodium, benzalkonium chloride and olopatadine HCl.
3. Add equivalent to approximately 0.55 g, 6N hydrochloric acid per 100 ml batch.
4. Allow adequate time between each addition for dissolution of each ingredient.
5. Add purified water to approximately 90% of final batch weight.
6. Measure pH and adjust, if necessary, to 3.7 with 6N (and/or 1 N) hydrochloric acid and 1 N sodium hydroxide.
7. Adjust to final batch weight with purified water (QS).
8. Measure final pH.
9. Filter through 0.2 µm filtration membrane.

Unexpectedly, it has been found that administration of relatively lower amounts of olopatadine to children results in more desirable relief of allergy symptoms than higher doses of olopatadine. As such, the present invention provides a particular dosing regimen, that can be used to achieve desired allergy relief. As used herein, the term "child" or "children" preferably includes only individuals less than 12 year of age and even more preferably includes only individuals that are at least 2 and more preferably at least 6 years of age but are no more than 11 years of age.

According to the regimen, a first amount of olopatadine is administered to the nostril of a child during a first time period. Then, during a second time period, a second amount of olopatadine is delivered to the nostrils of that child. The first amount and second amount of olopatadine are typically similar or substantially equivalent to each other. The first and second amounts are typically at least about 0.9 milligrams (mg) and more typically at least about 1.1 mg. The first and second amounts are typically no greater than about 1.5 mg and even more typically no greater than about 1.3 mg. The time periods for delivery of the first and second amounts of olopatadine (i.e., the first and second times periods during which the amounts are actually administered to the nostrils) are typically less than two minutes and more typically less that one minute. It is contemplated that various techniques can be employed to deliver the first and second amounts of olopatadine. However, it is preferable for these amounts to be administered using a nasal sprayer to deliver a single spray of a nasal spray solution to each nostril of the child within the prescribed amount of time. Preferred nasal spray solutions and nasal sprayer have been described herein.

Administration in accordance with the present invention also requires that a particular amount of time be allowed to pass between administration of the first amount and the second amount of olopatadine (i.e., between the first time period and the second time period). This intermediate time period is typically at least four hours and more typically at least eight hours. This intermediate time period is also typically less than twenty four hours and more typically less than sixteen hours. This intermediate time period can typically be easily achieved by administering the first amount in the morning and the second amount in the evening. The first amount and second amount of olopatadine can each be administered on a daily basis (i.e., the first amount and second amount can each be administered once in consecutive 24 hour periods) for multiple days (e.g., at least 15, 30, 45 or more days). For example, the amounts can be delivered daily through an allergy season.

The present invention also includes a pharmaceutical product for delivery of the olopatadine in accordance with the preferred dosing regimen. Such a product will typically include a nasal sprayer as described herein that contains an olopatadine nasal spray solution as described herein. The product will also typically include instructions for administration of the nasal spray solution associated with the nasal sprayer. Such instructions may be attached (e.g., adhered) directly to the nasal sprayer or may be provided with the nasal sprayer as part of a package (e:g., provided within a bag or box or attached to a bag or box in which the nasal sprayer is provided). Administration of the olopatadine nasal spray solution in accordance with these instructions will result in the desired first amount and second amount of olopatadine being delivered to the nostrils of a child in accordance with the present invention. For example, instructions to administer a single spray of olopatadine nasal solution having the formulation of Example 1 to each nostril of a child once in the morning and once in the evening will result in administration of the proper first and second amounts of olopatadine being deliver at proper times relative to each other.

### Study Results

As suggested earlier, administration of the relatively lower amounts of olopatadine according to the regimen described herein provides unexpectedly better efficacy in decreasing allergy symptoms relative to administration of olopatadine administered in relatively higher amounts. In a clinical study, children were divided into a first group and a second group. The first group received a single spray per nostril each day in the morning and the evening of either olopatadine nasal spray solution according to example 1 or of placebo. The second group received two sprays per nostril each day in the morning and the evening of either olopatadine nasal spray solution according to example 1 or of placebo for a period of at least 14 days and up to 24 days.

Each of the children in both the first group and the second group was then asked a series of questions.related to their allergy symptoms. In particular, each child of each group was asked, amongst other questions, whether they experienced reflective or instantaneous relief from allergy symptoms including stuffy nose, runny nose, itchy nose and sneezing. The questions related to reflective relief asked whether the patient experience relief from a particular symptom since their last symptom assessment. The questions related to instantaneous relief asked whether the patient was feeling relief from their symptoms at the time of the question.

The responses of the groups of children were statistically analyzed to determine whether administration of the olopatadine nasal spray solution exhibited statistically significant superiority in ameliorating the allergy symptoms relative to placebo. The results are provided below in table 2:

**TABLE 2**

| Efficacy Parameter | Superiority of Olopatadine Nasal Spray, 2.4 mg/day, (one spray per nostril in the morning and evening) compared to placebo nasal spray | Superiority of Olopatadine Nasal Spray, 4.8 mg/day, (two sprays per nostril in the morning and evening) compared to placebo nasal spray |
|---|---|---|
| Reflective itchy nose | Yes | Yes |
| Instantaneous itchy nose | Yes | No |
| Reflective sneezing | Yes | No |
| Instantaneous sneezing | Yes | No |
| Reflective runny nose | Yes | No |
| Instantaneous runny nose | Yes | No |
| Reflective stuffy nose | No | No |
| Instantaneous stuffy nose | Yes | No |
| Superiority on various efficacy parameters | 7 | 1 |

In table 2, a "yes" suggests that a statistically significant number of children felt that administration of olopatadine nasal spray at the dose indicated in the column in which the "yes" resides provided relief of the symptom listed in the row in which the "yes" resides. A "no", however, suggests that such is not the case. As can be seen, a statistically significant difference was more frequent for the lower dose of olopatadine nasal spray.

Statistical significance was determined using a p value of 0.05 (i.e., a 5% chance of obtaining the outcome observed assuming the null hypothesis is true) as the dividing line between statistical significant and statistically insignificant outcomes. Thus, an outcome showing a p value less than 0.05 is considered statistically significant while outcomes showing a p value greater than 0.05 are considered statistically insignificant. To further illustrate the statistical significance of the ability of the lower dose of olopatadine nasal spray to reduce allergy nasal symptoms, Table 3 is provided below:

**TABLE 3**

| | **Stuffy nose** | | **Runny nose** | | **Itchy nose** | | **Sneezing** | |
|---|---|---|---|---|---|---|---|---|
| | refl | inst | refl | inst | refl | inst | refl | lnst |
| Olo 0.6% 1 spray | -17.57 | -16.57 | -23.18 | -17.89 | -26.76 | -21.69 | -30.25 | -27.38 |
| Veh 1 spray | -14.93 | -10.64 | -16.37 | -7.63 | -15.84 | -9.89 | -17.73 | -10.56 |
| p-value | 0.2505 | 0.0144 | 0.0039 | 0.0159 | 0.0012 | 0.0019 | 0.0003 | 0.0001 |
| Olo 0.6% 2 sprays | -22.14 | -16.76 | -24.9 | -20.98 | -27.73 | -22.05 | -26.36 | -24.33 |
| Veh 2 sprays | -18.87 | -17.11 | -19.74 | -15.59 | -19.2 | -15.41 | -21.34 | -15.04 |
| p-value | 0.1762 | 0.8086 | 0.0824 | 0.1597 | 0.0048 | 0.0527 | 0.2828 | 0.1404 |

As can be seen, the lower dose of olopatadine nasal spray statistically exhibited a relatively high degree of superiority over placebo relative to the higher dose of olopatadine nasal.spray.

## Claims

1. Olopatadine nasal spray for use in a method of administering the spray, comprising:
administering a first amount of olopatadine to nostrils of a child during a first time period; and
administering a second amount of olopatadine to the nostrils of the child during a second time period;
wherein:
i) the first amount and second amount are each at least about 0.9 mg but is no more than about 1.5 mg;
ii) the first time period and second time period are less than two minutes;
iii) the first time period and the second time period are separated by an intermediate time period that is at least four hours but is less than twenty four hours; and
iv) the child is at least 2 years of age but less than 12 years of age.

2. Olopatadine nasal spray as in claim 1 wherein the first amount and the second amount are administered from a nasal spray bottle to the nostrils.

3. Olopatadine nasal spray as in claim 2 wherein the first amount and second amount are each delivered to the nostrils of the child using a single spray from the nasal spray bottle in each nostril.

4. Olopatadine nasal spray as in claim 1, 2 or 3 wherein the first amount and second amount are both at least about 1.1 mg.

5. Olopatadine nasal spray as in claim 1, 2, 3 or 4 wherein the first amount and second amount are both no more than about 1.3 mg.

6. Olopatadine nasal spray as in any of claims 1-5 wherein the intermediate time period is at least about 8 hours.

7. Olopatadine nasal spray as in any of claims 1-6 wherein the intermediate time period is no more than about 16 hours.

8. Olopatadine nasal spray as in any of claims 1-7 wherein the first amount and second amount of olopatadine are administered as part of a composition and the composition consists essentially of:
a) 0.54 - 0.62 % (w/v) olopatadine free base or an equivalent amount of a pharmaceutically acceptable salt of olopatadine;
b) a phosphate salt in an amount equivalent to 0.2 - 0.8 % (w/v) dibasic sodium phosphate, wherein the phosphate salt selected from the group consisting of monobasic sodium phosphate; dibasic sodium phosphate; tribasic sodium phosphate; monobasic potassium phosphate; dibasic potassium phosphate; and tribasic potassium phosphate;
c) 0.3 - 0.6 % (w/v) NaCl;
d) a pH-adjusting agent in an amount sufficient to cause the composition to have a pH of 3.6 - 3.8;
e) 0.005 - 0.015 % (w/v) benzalkonium chloride;
f) 0.005 - 0.015 % (w/v) edetate disodium; and
g) water.

9. Olopatadine nasal spray as in any of claims 1-8 wherein the first amount and second amount of olopatadine are administered as part of a composition and the composition consists essentially of:
a) 0.6 % (w/v) olopatadine free base or an equivalent amount of a pharmaceutically acceptable salt of olopatadine;
b) 0.4 - 0.6 % (w/v) dibasic sodium phosphate;
c) 0.35 - 0.45 % (w/v) NaCl;
d) a pH-adjusting agent in an amount sufficient to cause the composition to have a pH of 3.6 - 3.8, wherein the pH-adjusting agent is selected from the group consisting of NaOH and HCl;
e) 0.01 % (w/v) benzalkonium chloride;
f) 0.01 % (w/v) edetate disodium; and
g) water.

10. An olopatadine pharmaceutical product, comprising:
a nasal sprayer containing an olopatadine nasal spray according to claim 1; and
instructions for administration of the nasal spray to a child provided with the nasal sprayer wherein administration of the nasal spray according to the instructions would result in administration of olopatadine in accordance with the following regimen:
i) administration of a first amount of olopatadine to nostrils of a child during a first time period wherein the first time period is less than two minutes and wherein the child is at least 2 years of age but less than 12 years of age and wherein the first amount is at least about 0.9 mg but is no more than about 1.5 mg; and
ii) administration of a second amount of olopatadine to the nostrils of the child during a second time period wherein the second time period is less than two minutes and wherein the first time period and the second time period are separated by an intermediate time period that is at least four hours but is less than twenty four hours and the second time period is less than two minutes and wherein the second amount is at least about 0.9 mg but is no more than about 1.5 mg.

11. A product as in claim 10 wherein the first amount and second amount are each delivered to the nostrils of the child using a single spray from the nasal spray bottle in each nostril.

12. A product as in claim 10 or 11 wherein the first amount and second amount are both at least about 1.1 mg.

13. A product as in claim 10, 11 or 12 wherein the first amount and second amount are both no more than about 1.3 mg.

14. A product as in any of claims 10-13 wherein the intermediate time period is at least about 8 hours.

15. A product as in any of claims 10-14 wherein the intermediate time period is no more than about 16 hours.

16. A product as in any of claims 10-15 wherein the first amount and second amount of olopatadine are administered as part of a composition and the composition consists essentially of:
a) 0.54 - 0.62 % (w/v) olopatadine free base or an equivalent amount of a pharmaceutically acceptable salt of olopatadine;
b) a phosphate salt in an amount equivalent to 0.2 - 0.8 % (w/v) dibasic sodium phosphate, wherein the phosphate salt selected from the group consisting of monobasic sodium phosphate; dibasic sodium phosphate; tribasic sodium phosphate; monobasic potassium phosphate; dibasic potassium phosphate; and tribasic potassium phosphate;
c) 0.3 - 0.6 % (w/v) NaCl;
d) a pH-adjusting agent in an amount sufficient to cause the composition to have a pH of 3.6 - 3.8;
e) 0.005 - 0.015 % (w/v) benzalkonium chloride;
f) 0.005 - 0.015 % (w/v) edetate disodium; and
g) water.

17. A product as in any of claims 10-16 wherein the first amount and second amount of olopatadine are administered as part of a composition and the composition consists essentially of:
a) 0.6 % (w/v) olopatadine free base or an equivalent amount of a pharmaceutically acceptable salt of olopatadine;
b) 0.4 - 0.6 % (w/v) dibasic sodium phosphate;
c) 0.35 - 0.45 % (w/v) NaCl;
d) a pH-adjusting agent in an amount sufficient to cause the composition to have a pH of 3.6 - 3.8, wherein the pH-adjusting agent is selected from the group consisting of NaOH and HCl;
e) 0.01 % (w/v) benzalkonium chloride;
f) 0.01 % (w/v) edetate disodium; and
g) water.

18. A product as in any of the preceding claims wherein the first amount and second amount of olopatadine are each repeatedly administered on a daily basis for multiple days.

19. A product as in any of the preceding claims wherein the child is at least 6 years of age.

## Patentansprüche

1. Olopatadin Nasenspray zur Verwendung in einem Verfahren zur Verabreichung des Sprays umfassend:
Verabreichen einer ersten Menge an Olopatadin in die Nasenlöcher eines Kindes während eines ersten Zeitraums; und
Verabreichen einer zweiten Menge an Olopatadin in die Nasenlöcher eines Kindes während eines zweiten Zeitraums;
wobei:
i) sowohl die erste Menge als auch die zweite Menge wenigstens etwa 0,9 mg, jedoch nicht mehr als etwa 1,5 mg beträgt;
ii) der erste Zeitraum und der zweite Zeitraum weniger als zwei Minuten beträgt;
iii) der erste Zeitraum und der zweite Zeitraum durch einen Zwischenzeitraum getrennt sind, der wenigstens vier Stunden, jedoch weniger als vierundzwanzig Stunden beträgt; und
iv) das Kind wenigstens 2 Jahre, jedoch weniger als 12 Jahre alt ist.

2. Olopatadin Nasenspray wie in Anspruch 1, wobei die erste Menge und die zweite Menge aus einer Nasensprayflasche in die Nasenlöcher verabreicht werden.

3. Olopatadin Nasenspray wie in Anspruch 2, wobei die erste Menge und die zweite Menge jeweils in die Nasenlöcher des Kindes unter Verwendung eines einzigen Sprühstoßes aus der Nasensprayflasche in jedes Nasenloch abgegeben werden.

4. Olopatadin Nasenspray wie in Anspruch 1, 2 oder 3, wobei sowohl die erste Menge als auch die zweite Menge wenigstens etwa 1,1 mg betragen.

5. Olopatadin Nasenspray wie in Anspruch 1, 2, 3 oder 4, wobei sowohl die erste Menge als auch die zweite Menge nicht mehr als etwa 1,3 mg betragen.

6. Olopatadin Nasenspray wie in einem der Ansprüche 1-5, wobei der Zwischenzeitraum wenigstens etwa 8 Stunden beträgt.

7. Olopatadin Nasenspray wie in einem der Ansprüche 1-6, wobei der Zwischenzeitraum nicht mehr als etwa 16 Stunden beträgt.

8. Olopatadin Nasenspray wie in einem der Ansprüche 1-7, wobei die erste Menge und die zweite Menge an Olopatadin als Teil einer Zusammensetzung verabreicht werden und die Zusammensetzung im Wesentlichen aus:
a) 0,54 - 0,62 % (G/V) Olopatadin als freie Base oder eine äquivalente Menge eines pharmazeutisch verträglichen Salzes von Olopatadin;
b) ein Phosphatsalz in einer Menge, die äquivalent ist zu 0,2 - 0,8 % (G/V) Dinatriumphosphat, wobei das Phosphatsalz ausgewählt ist aus der Gruppe bestehend aus Mononatriumphosphat; Dinatriumphosphat; Trinatriumphosphat; Monokaliumphosphat, Dikaliumphosphat; und Trikaliumphosphat;
c) 0,3 - 0,6 % (G/V) NaCl;
d) einem Mittel zur Einstellung des pH-Wertes in einer ausreichenden Menge, um die Zusammensetzung auf einen pH-Wert von 3,6 -3,8 einzustellen;
e) 0,005 -0,015 % (G/V) Benzalkoniumchlorid
f) 0,005 - 0,01 % /G/V) Dinatriumedetat; und
g) Wasser
besteht.

9. Olopatadin Nasenspray, wie in einem der Ansprüche 1-8, wobei die erste Menge und die zweite Menge an Olopatadin als Teil einer Zusammensetzung verabreicht werden und die Zusammensetzung im Wesentlichen aus:
a) 0,6 % (G/V) Olopatadin als freie Base oder eine äquivalente Menge eines pharmazeutisch verträglichen Salzes von Olopatadin;
b) 0,4 - 0,6 % (G/V) Dinatriumphosphat (zwei-basisches Natriumphosphat);
c) 0,35 - 0,45 % (G/V) NaCl;
d) einem Mittel zur Einstellung des pH-Wertes in einer ausreichenden Menge, um die Zusammensetzung auf einen p-Wert von 3,6 - 3,8 einzustellen, wobei das Mittel zur Einstellung des pH-Wertes ausgewählt ist aus der Gruppe bestehend aus NaOH und HCl;
e) 0,01 % (G/V) Benzalkoniumchlorid;
f) 0,01 % (GN) Dinatriumedetat; und
g) Wasser
besteht.

10. Olopatadin Arzneimittel umfassend:
einen Nasensprüher, der einen Olopatadinnasenspray nach Anspruch 1 enthält und Anweisungen zur Verabreichung des Nasensprays an ein Kind, die mit dem Nasensprüher zur Verfügung gestellt werden, wobei die Verabreichung des Nasensprays gemäß den Anweisungen in der Verabreichung von Olopatadin gemäß dem folgenden Regim resultieren würde:
i) Verabreichen einer ersten Menge an Olopatadin in die Nasenlöcher eines Kindes während eines ersten Zeitraums, wobei der erste Zeitraum weniger als zwei Minuten beträgt und wobei das Kind wenigstens 2 Jahre, jedoch weniger als 12 Jahre alt ist und wobei die erste Menge wenigstens 0,9 mg, jedoch nicht mehr als etwa 1,5 mg beträgt; und
ii) Verabreichen einer zweiten Menge an Olopatadin in die Nasenlöcher des Kindes während eines zweiten Zeitraums, wobei der zweite Zeitraum weniger als zwei Minuten beträgt und wobei der erste Zeitraum und der zweite Zeitraum durch einen Zwischenzeitraum getrennt sind, der wenigstens vier Stunden, jedoch nicht weniger als vierundzwanzig Stunden beträgt und der zweite Zeitraum weniger als zwei Minuten beträgt und wobei die zweite Menge wenigstens etwa 0,9 mg, jedoch nicht mehr als etwa 1,5 mg beträgt.

11. Arzneimittel wie in Anspruch 10, wobei sowohl die erste Menge als auch die zweite Menge jeweils in die Nasenlöcher des Kindes unter Verwendung eines einzigen Sprühstoßes aus der Nasensprayflasche in jedes Nasenloch abgegeben werden.

12. Arzneimittel wie in Anspruch 10 oder 11, wobei sowohl die erste Menge als auch die zweite Menge wenigstens etwa 1,1 mg betragen.

13. Arzneimittel wie in Anspruch 10, 11 oder 12, wobei sowohl die erste Menge als auch die zweite Menge nicht mehr als etwa 1,3 mg betragen.

14. Arzneimittel wie in einem der Ansprüche 10-13, wobei der Zwischenzeitraum wenigstens etwa 8 Stunden beträgt.

15. Arzneimittel wie in einem der Ansprüche 10-14, wobei der Zwischenzeitraum nicht mehr als etwa 16 Stunden beträgt.

16. Arzneimittel wie in einem der Ansprüche 10-15, wobei die erste Menge und die zweite Menge an Olopatadin als Teil einer Zusammensetzung verabreicht werden und die Zusammensetzung im Wesentlichen aus:
a) 0,54 - 0,62 % (GN) Olopatadin als freie Base oder eine äquivalente Menge eines pharmazeutisch verträglichen Salzes von Olopatadin;
b) ein Phosphatsalz in einer Menge, die äquivalent ist zu 0,2 - 0,8 % (GN) Dinatiumphosphat, wobei das Phosphatsalz ausgewählt ist aus der Gruppe bestehend aus Mononatriumphosphat; Dinatriumphosphat; Trinatriumphosphat; Monokaliumphosphat; Dikaliumphosphat; und Trikaliumphosphat;
c) 0,3 - 0,6 % (GN) NaCl;
d) ein Mittel zur Einstellung des pH-Wertes in einer ausreichenden Menge um die Zusammensetzung auf einen pH-Wert von 3,6 - 3,8 einzustellen;
e) 0,005 - 0,015 % (GN) Benzalkoniumchlorid;
f) 0,005 - 0,015 % (GN) Dinatriumedetat; und
g) Wasser
besteht.

17. Arzneimittel wie in einem der Ansprüche 10-16, wobei die erste Menge und die zweite Menge an Olopatadin als Teil einer Zusammensetzung verabreicht werden und die Zusammensetzung im Wesentlichen aus:
a) 0,6 % (GN) Olopatadin als freie Base oder eine äquivalente Menge eines pharmazeutisch verträglichen Salzes von Olopatadin;
b) 0,4 - 0,6 % (G/V) Dinatiumphosphat (zwei-basisches Natriumphosphat);
c) 0,35 - 0,45 % (G/V) NaCl;
d) ein Mittel zur Einstellung das pH-Wertes in einer ausreichenden Menge, um die Zusammensetzung auf einen pH-Wert von 3,6 - 3,8 einzustellen, wobei das Mittel zur Einstellung des pH-Wertes ausgewählt ist aus der Gruppe bestehend aus NaOH und HCl;
e) 0,01 % (G/V) Benzalkoniumchlorid;
f) 0,01 % (G/V) Dinatriumedetat; und
g) Wasser
besteht.

18. Arzneimittel wie in einem der vorstehenden Ansprüche, wobei sowohl die erste Menge als auch die zweite Menge an Olopatadin jeweils wiederholt tageweise über mehrere Tage verabreicht werden.

19. Arzneimittel wie in einem der vorstehenden Ansprüche, wobei das Kind wenigstens 6 Jahre alt ist.

## Revendications

1. Pulvérisation nasale à base d'olopatadine destinée à être utilisée dans un procédé d'administration de la pulvérisation, comprenant :
l'administration d'une première quantité d'olopatadine dans les narines d'un enfant pendant une première période de temps ; et
l'administration d'une seconde quantité d'olopatadine dans les narines de l'enfant pendant une seconde période de temps ;
dans laquelle :
i) la première quantité et la seconde quantité sont chacune d'au moins environ 0,9 mg, mais ne sont pas supérieures à environ 1,5 mg ;
ii) la première période de temps et la seconde période de temps sont inférieures à deux minutes ;
iii) la première période de temps et la seconde période de temps sont séparées par une période de temps intermédiaire qui est d'au moins quatre heures, mais qui est inférieure à vingt-quatre heures ; et
iv) l'enfant est âgé d'au moins 2 ans, mais de moins de 12 ans.

2. Pulvérisation nasale à base d'olopatadine selon la revendication 1, dans laquelle la première quantité et la seconde quantité sont administrées à partir d'une bouteille de pulvérisation nasale dans les narines.

3. Pulvérisation nasale à base d'olopatadine selon la revendication 2, dans laquelle la première quantité et la seconde quantité sont chacune délivrées dans les narines de l'enfant en utilisant une pulvérisation unique de la bouteille de pulvérisation nasale dans chaque narine.

4. Pulvérisation nasale à base d'olopatadine selon la revendication 1, 2 ou 3, dans laquelle la première quantité et la seconde quantité sont toutes les deux d'au moins environ 1,1 mg.

5. Pulvérisation nasale à base d'olopatadine selon la revendication 1, 2, 3 ou 4, dans laquelle la première quantité et la seconde quantité sont toutes les deux inférieures ou égales à environ 1,3 mg.

6. Pulvérisation nasale à base d'olopatadine selon l'une quelconque des revendications 1 à 5, dans laquelle la période de temps intermédiaire est d'au moins environ 8 heures.

7. Pulvérisation nasale à base d'olopatadine selon l'une quelconque des revendications 1 à 6, dans laquelle la période de temps intermédiaire n'est pas supérieure à environ 16 heures.

8. Pulvérisation nasale à base d'olopatadine selon l'une quelconque des revendications 1 à 7, dans laquelle la première quantité et la seconde quantité d'olopatadine sont administrées dans le cadre d'une composition, et la composition est essentiellement constituée par :
a) 0,54 à 0,62 % (p/v) d'une base libre d'olopatadine ou d'une quantité équivalente d'un sel pharmaceutiquement acceptable d'olopatadine ;
b) un sel de phosphate en une quantité équivalente à 0,2 à 0,8 % (p/v) de phosphate de sodium dibasique, le sel de phosphate étant choisi dans le groupe constitué par le phosphate de sodium monobasique ; le phosphate de sodium dibasique ; le phosphate de sodium tribasique ; le phosphate de potassium monobasique ; le phosphate de potassium dibasique ; et le phosphate de potassium tribasique ;
c) 0,3 à 0,6 % (p/v) de NaCl ;
d) un agent d'ajustement du pH en une quantité suffisante pour que la composition ait un pH de 3,6 à 3,8 ;
e) 0,005 à 0,015 % (p/v) de chlorure de benzalkonium ;
f) 0,005 à 0,015 % (p/v) d'édétate disodique ; et
g) de l'eau.

9. Pulvérisation nasale à base d'olopatadine selon l'une quelconque des revendications 1 à 8, dans laquelle la première quantité et la seconde quantité d'olopatadine sont administrées dans le cadre d'une composition, et la composition est essentiellement constituée par :
a) 0,6 % (p/v) d'une base libre d'olopatadine ou d'une quantité équivalente d'un sel pharmaceutiquement acceptable d'olopatadine ;
b) 0,4 à 0,6 % (p/v) de phosphate de sodium dibasique ;
c) 0,35 à 0,45 % (p/v) de NaCl ;
d) un agent d'ajustement du pH en une quantité suffisante pour que la composition ait un pH de 3,6 à 3,8, l'agent d'ajustement du pH étant choisi dans le groupe constitué par NaOH et HCl ;
e) 0,01 % (p/v) de chlorure de benzalkonium ;
f) 0,01 % (p/v) d'édétate disodique ; et
g) de l'eau.

10. Produit pharmaceutique à base d'olopatadine, comprenant :
un pulvérisateur nasal contenant une pulvérisation nasale à base d'olopatadine selon la revendication 1 ; et
des instructions pour l'administration de la pulvérisation nasale à un enfant à l'aide du pulvérisateur nasal, l'administration de la pulvérisation nasale selon les instructions résultant en l'administration d'olopatadine selon le régime suivant :
i) l'administration d'une première quantité d'olopatadine dans les narines d'un enfant pendant une première période de temps, la première période de temps étant inférieure à deux minutes et l'enfant étant âgé d'au moins 2 ans, mais de moins de 12 ans, et la première quantité étant d'au moins environ 0,9 mg, mais n'étant pas supérieure à environ 1,5 mg ; et
ii) l'administration d'une seconde quantité d'olopatadine dans les narines de l'enfant pendant une seconde période de temps, la seconde période de temps étant inférieure à deux minutes et la première période de temps et la seconde période de temps étant séparées par une période de temps intermédiaire qui est d'au moins quatre heures mais qui est inférieure à vingt-quatre heures, et la seconde période de temps étant inférieure à deux minutes, et la seconde quantité étant d'au moins environ 0,9 mg, mais n'étant pas supérieure à environ 1,5 mg.

11. Produit selon la revendication 10, dans lequel la première quantité et la seconde quantité sont chacune délivrées dans les narines de l'enfant en utilisant une pulvérisation unique de la bouteille de pulvérisation nasale dans chaque narine.

12. Produit selon la revendication 10 ou 11, dans lequel la première quantité et la seconde quantité sont toutes les deux d'au moins environ 1,1 mg.

13. Produit selon la revendication 10, 11 ou 12, dans lequel la première quantité et la seconde quantité sont toutes les deux inférieures ou égales à environ 1,3 mg.

14. Produit selon l'une quelconque des revendications 10 à 13, dans lequel la période de temps intermédiaire est d'au moins environ 8 heures.

15. Produit selon l'une quelconque des revendications 10 à 14, dans lequel la période de temps intermédiaire n'est pas supérieure à environ 16 heures.

16. Produit selon l'une quelconque des revendications 10 à 15, dans lequel la première quantité et la seconde quantité d'olopatadine sont administrées dans le cadre d'une composition, et la composition est essentiellement constituée par :
a) 0,54 à 0,62 % (p/v) d'une base libre d'olopatadine ou d'une quantité équivalente d'un sel pharmaceutiquement acceptable d'olopatadine ;
b) un sel de phosphate en une quantité équivalente à 0,2 à 0,8 % (p/v) de phosphate de sodium dibasique, le sel de phosphate étant choisi dans le groupe constitué par le phosphate de sodium monobasique ; le phosphate de sodium dibasique ; le phosphate de sodium tribasique ; le phosphate de potassium monobasique ; le phosphate de potassium dibasique ; et le phosphate de potassium tribasique ;
c) 0,3 à 0,6 % (p/v) de NaCl ;
d) un agent d'ajustement du pH en une quantité suffisante pour que la composition ait un pH de 3,6 à 3,8 ;
e) 0,005 à 0,015 % (p/v) de chlorure de benzalkonium ;
f) 0,005 à 0,015 % (p/v) d'édétate disodique ; et
g) de l'eau.

17. Produit selon l'une quelconque des revendications 10 à 16, dans lequel la première quantité et la seconde quantité d'olopatadine sont administrées dans le cadre d'une composition, et la composition est essentiellement constituée par :
a) 0,6 % (p/v) d'une base libre d'olopatadine ou d'une quantité équivalente d'un sel pharmaceutiquement acceptable d'olopatadine ;
b) 0,4 à 0,6 % (p/v) de phosphate de sodium dibasique ;
c) 0,35 à 0,45 % (p/v) de NaCl ;
d) un agent d'ajustement du pH en une quantité suffisante pour que la composition ait un pH de 3,6 à 3,8, l'agent d'ajustement du pH étant choisi dans le groupe constitué par NaOH et HCl ;
e) 0,01 % (p/v) de chlorure de benzalkonium ;
f) 0,01 % (p/v) d'édétate disodique ; et
g) de l'eau.

18. Produit selon l'une quelconque des revendications précédentes, dans lequel la première quantité et la seconde quantité d'olopatadine sont chacune administrées de manière répétée quotidiennement pendant plusieurs jours.

19. Produit selon l'une quelconque des revendications précédentes, dans lequel l'enfant est âgé d'au moins 6 ans.
